# EUROPEAN PATENT SPECIFICATION

(11) **EP 4 104 885 B1**
(45) Date of publication and mention of the grant of the patent: **28.02.2024**
(21) Application number: 22177020.9
(22) Date of filing: 02.06.2022
(51) Int. Cl.: A61M 16/00, A63B 23/18, A61M 16/20

(54) **OSCILLATING POSITIVE EXPIRATORY PRESSURE DEVICE**
SCHWINGENDE VORRICHTUNG FÜR POSITIVEN EXSPIRATORISCHEN DRUCK
DISPOSITIF DE PRESSION EXPIRATOIRE POSITIVE OSCILLANTE

(30) Priority: 15.06.2021 TW 110121639
(43) Date of publication of application: 21.12.2022
(73) Proprietor: Enchant Tek. Co., Ltd., Dongshan Township 26950 (TW)
(72) Inventor: Chen, Chun-Hung, Dongshan Township 26950 (TW); Lin, Ling-Ling Leonard, Dongshan Township 26950 (TW); Huang, Min-Hsien, Dongshan Township 26950 (TW)
(74) Representative: Taruttis, Tilman

(56) References cited:
- EP-A2- 1 103 287
- WO-A1-2015/118482
- US-A1- 2006 090 753
- US-A1- 2018 256 839

## Description

The disclosure relates to a positive expiratory pressure (PEP) device, more particularly to an oscillating positive expiratory pressure (OPEP) device.

As air pollution has worsened in many places around the world, environmental allergens have increased considerably, making modern people more susceptible to respiratory disorders including cystic fibrosis (CF) and chronic obstructive pulmonary disease (COPD), which cause excessive secretions in their lungs and tracheas. Coughing should loosen secretions and subsequently expel them from the trachea of an average healthy person. In those with respiratory problems, however, a single cough may be unlikely to dislodge an obstruction in the trachea. In these cases, medication and oscillating positive expiratory pressure (OPEP) therapy are used to treat secretion accumulation. OPEP therapy uses a conventional OPEP device (such as an Acapella device) to open clogged tracheas and loosen secretions by oscillating vibrations of expiratory air pressure during the expiration of a user, allowing the user to cough them out and clear lung obstruction.

The conventional OPEP device, which controls the user's expiration resistance using a magnet, is currently used for therapeutic treatment. An example for an OPEP device using a magnet to control the user's expiration can be found in US 2006/0090753 A1. However, the structure of this device is relatively complex and bulky, and it isn't easy to clean.

Therefore, the object of the disclosure is to provide an oscillating positive expiratory pressure device that can alleviate at least one of the drawbacks associated with the abovementioned prior art.

An oscillating positive expiratory pressure device includes a housing, a top cover, and an oscillating unit. The housing includes a bottom wall and a surrounding wall that extends upwardly from the bottom wall. The bottom wall has an inclined enclosing surface that extends downwardly and that terminates at an opening. The top cover is connected to the surrounding wall, and cooperates with the bottom wall and the surrounding wall to define an accommodating space. The oscillating unit is swingably connected to the housing and is disposed within the accommodating space. The oscillating unit includes a swing member, a first weighting piece, and a second weighting piece. The swing member includes a swing arm, a first swing block connected to one end of the swing arm, and a second swing block connected to another end of the swing arm and adjacent to the opening. The first weighting piece is carried on the first swing block. The second weighting piece is carried on the second swing block. The swing arm is swingable to move the second swing block to block and unblock the opening. The first swing block is configured and adapted to carry said first weighting piece of variable weights and the second swing block is configured and adapted to carry said second weighting piece of variable weights to change an oscillating frequency of said oscillating unit.

Other features and advantages of the disclosure will become apparent in the following detailed description of the embodiment with reference to the accompanying drawings, of which:
Figure 1 is a perspective view of an embodiment of an oscillating positive expiratory pressure device according to the disclosure;
Figure 2 is a partly exploded perspective view of the embodiment;
Figure 3 is a partly exploded perspective view of the embodiment from another perspective;
Figure 4 is a cross-sectional view taken along line IV-IV in Figure 1, illustrating an oscillating unit in a closed position; and
Figure 5 is a cross-sectional view similar to Figure 4, illustrating the oscillating unit in an open position.

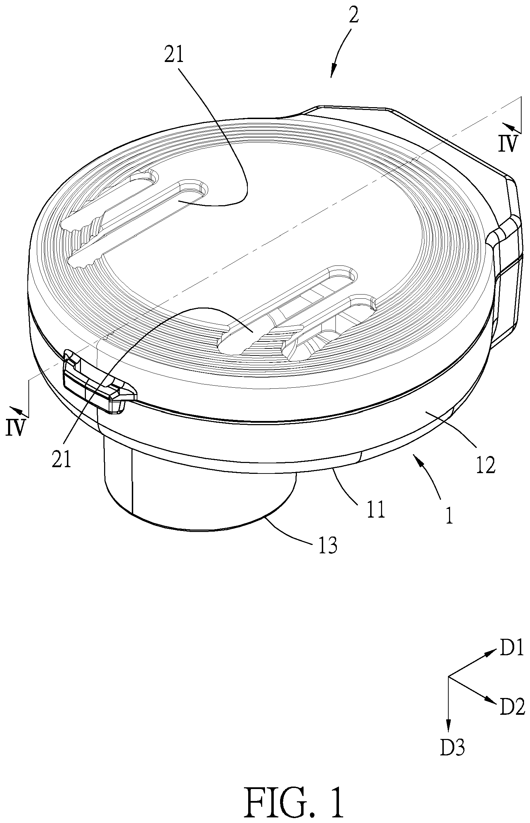

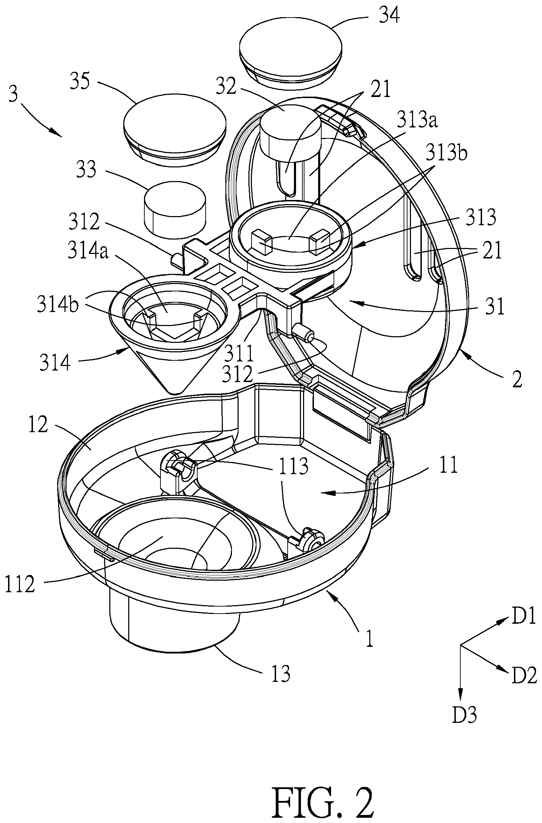

As shown in Figures 1 and 2, the embodiment of the oscillating positive expiratory pressure device as claimed in the present disclosure can be used to help a user in loosening secretions attached to the tracheas so that the user can then cough them out. The oscillating positive expiratory pressure device includes a housing 1, a top cover 2 and an oscillating unit 3.

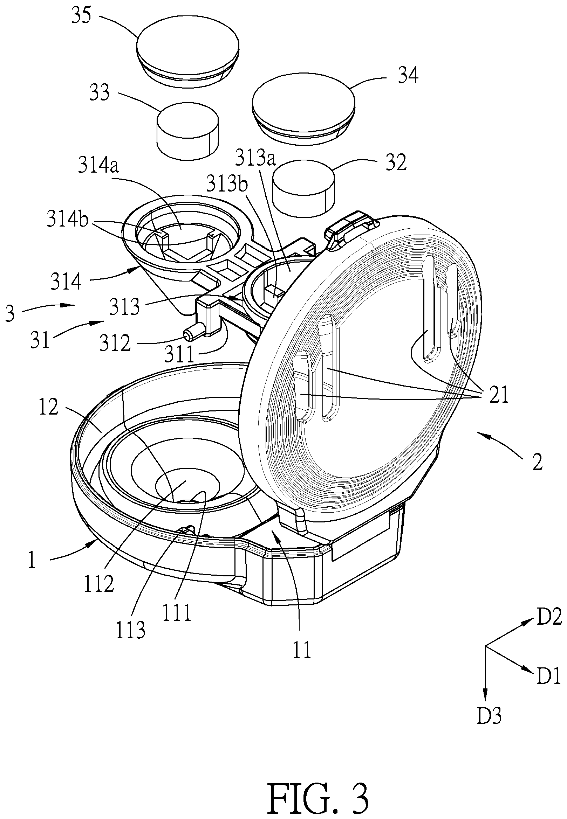

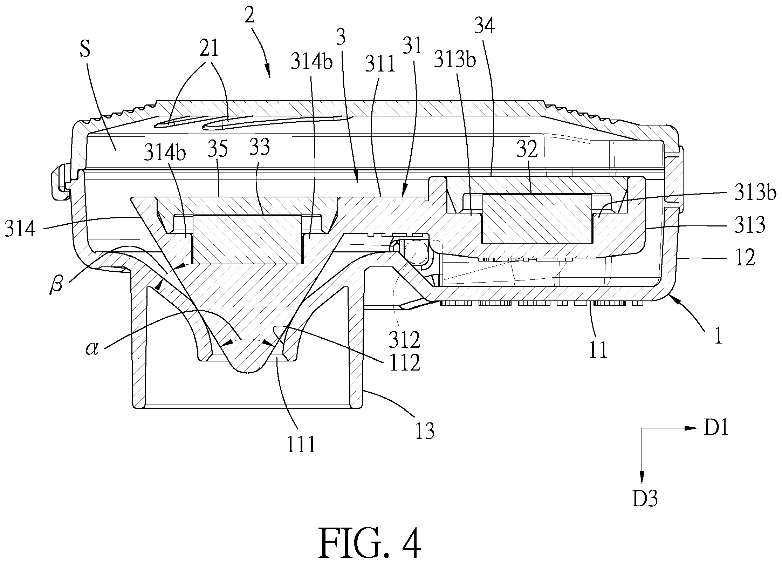

Referring to Figures 2 to 4, the housing 1 includes a bottom wall 11, a surrounding wall 12 extending upward from the bottom wall 11 along an up-down direction (D3), and an input tube 13 extending downwardly from the bottom wall 11 along the up-down direction (D3). The bottom wall 11 has an inclined enclosing surface 112 that is substantially funnel-shaped, that extends downwardly from the bottom wall 11, that terminates at an opening 111 and that is surrounded by the input tube 13. The bottom wall 11 further has two support brackets 113 extending upright. A cross-sectional width of the inclined enclosing surface 112 decreases in the up-down direction (D3), as shown in Figure 4. The inclined enclosing surface 112 has a curved portion adjacent to the opening 111. The oscillating unit 3 is swingably mounted on the support brackets 113 of the bottom wall 11. The top cover 2 is connected to the surrounding wall 12, and cooperates with the surrounding wall 12 and the bottom wall 11 to define an accommodating space (S). The top cover 2 is formed with four vent holes 21 extending along a front-rear direction (D1) and spaced apart from each other. The vent holes 21 are positioned corresponding to the opening 111. The total area of the vent holes 21 occupies less than 50% of the area of the top cover 2. In this embodiment, the total area of the vent holes 21 is less than 5 square centimeters, but the area is not limited to this figure in other embodiments. The number and extension direction of the vent holes 21 are also not limited to those mentioned above.

The oscillating unit 3 is disposed within the accommodating space (S), and includes a swing member 31 including a swing arm 311 that extends in the front-rear direction (D1), a first swing block 313 that is connected to one end of the swing arm 311, and a second swing block 314 that is connected to another end of the swing arm 311 and that is adjacent to the opening 111. The oscillating unit 3 further includes a first weighting piece 32 that is carried on the first swing block 313, a second weighting piece 33 that is carried on the second swing block 314, a first lid 34 that is disposed over the first weighting piece 32, and a second lid 35 that is disposed over the second weighting piece 33. and the swing member 31 further includes two rotary pins 312 protruding laterally and respectively from opposite sides of the swing arm 31 parallel to a left-right direction (D2) and being held respectively by the support brackets 113 to form a rotating axis of the swing member 31 so that the swing member 31 can swing about the rotating axis. The first swing block 313 is substantially cylindrical-shaped, and has a first accommodating groove 313a accommodating the first weighting piece 32 therein, and four first retaining ribs 313b disposed in the first accommodating groove 313a and abutting against the first weighting piece 32. The second swing block 314 is substantially conical and tapers toward the opening 111, and has a second accommodating groove 314a accommodating the second weighting piece 33 therein, and four second retaining ribs 314b disposed in the second accommodating groove 314a and abutting against the second weighting piece 33. The first lid 34 and the second lid 35 respectively close the first accommodating groove 313a and the second accommodating groove 314a. The first weighting piece 32 and the second weighting piece 33 can be positioned in a tightly fitted manner in the first accommodating groove 313a and the second accommodating groove 314a through the first retaining ribs 313b and the second retaining ribs 314b.

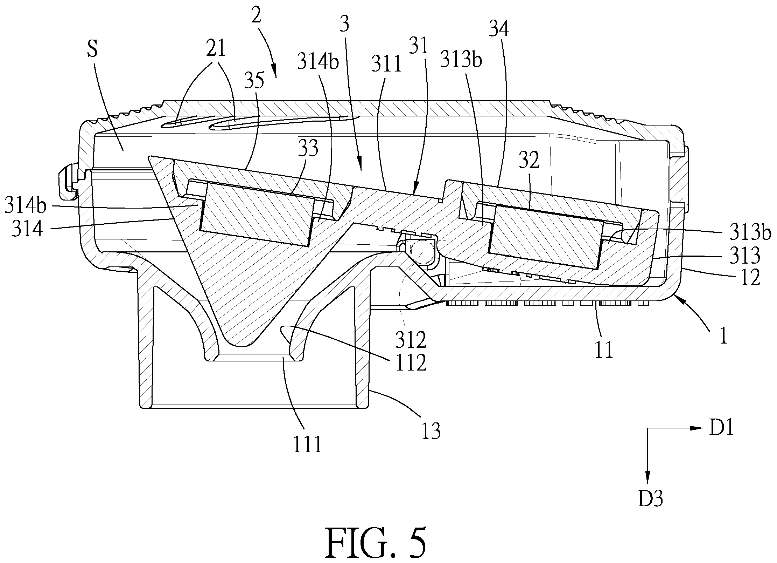

Referring to Figures 4 and 5, the oscillating unit 3 can swing about the rotary pins 312 relative to the bottom wall 11 of the housing 1, between closed and open positions. In the closed position, the second swing block 314 is inserted into the opening 111 with a lateral surface thereof being in contact with the inclined enclosing surface 112, thereby blocking the opening 111, as shown in Figure 4. The oscillating unit 3 can swing upwardly from the closed position to the open position. During the conversion of the oscillating unit 3 from the closed position to the open position, the first swing block 313 swings downwardly to contact the bottom wall 11, and the second swing block 314 swings upwardly to be separated from the inclined enclosing surface 112 and unblock the opening 111, as shown in Figure 5.

Each of the first weighting piece 32 and the second weighting piece 33 has a density greater than 2, and the preferred weight of the first and second weighting pieces 32, 32 ranges between 1 to 4 grams. To achieve a generally smooth oscillation of the oscillating unit 3, the first and second weighting pieces 32, 33 should preferably be of the same weight.

A cross section of the second swing block 314 along an axis of the second swing block 314 in the up-down direction (D3) has two opposite sides that form an included angle (α) therebetween. The included angle (α) affects the Coanda Effect at the inclined enclosing surface 112, and is preferably between 53 degrees and 73 degrees. Actual measurements show that an incorporated angle between 58 degrees and 68 degrees offers more desirable operational performance. On the other hand, when the oscillating unit 3 is in the closed position, an included angle (β) defined between each of the opposite sides of the cross section of the second swing block 314 along the axis of the second swing block 314 and the inclined enclosing surface 112 will affect the swing of the oscillating unit 3, and ranges between 10 and 30 degrees. More preferably the included angle (β) ranges between 16 to 24 degrees.

### Flow rate and weighting piece test

The aforementioned preferred angle setting is shown in Table 1. The test is carried out by simulating an average adult with an expiratory flow rate ranging from 5 to 30 liters per minute and a static expiratory pressure of 8 to 18 cmH₂O.It has been recognized by those skilled in the art that the most successful treatment is obtained when the pressure change of the expiratory airflow, corresponding to the amplitude of airflow oscillation, ranges between 5 to 20 cmH₂O, with a frequency of airflow oscillation between 10 to 40 Hz. A to D listed in the column head of Table 1 are the different settings of the included angle (β) for the present oscillating positive expiratory pressure device, which are 16.75 degrees, 20 degrees, 23.3 degrees, and 26.5 degrees in sequence. The different weights of the first and second weighting pieces 32 and 33 (unit: grams) are listed in each column of the second row labeled "weighting piece." The varying frequencies of airflow oscillation (Hz, oscillations per second) at different simulated expiratory flow rates (5 LPM to 30 LPM as listed in the left-hand column) are given in Table 1. Different frequencies of airflow oscillation will be achieved by varying different selected weights at different simulated expiratory flow rates, as given in Table 1. A lower oscillating frequency is more likely to be achieved by increasing in the selected weight of the weighting pieces. Table 2 depicts the varying amplitudes of the simulated user's expiratory airflow oscillation (unit: cmH₂O) obtained by varying the selected weight of the weighting pieces. It can be determined from the data in the table that the amplitude of the expiratory airflow oscillation is more likely to grow with an increase of the selected weight of the weighting piece.

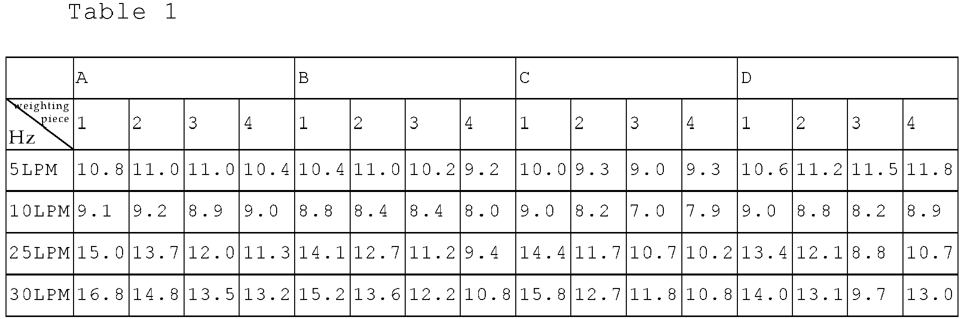

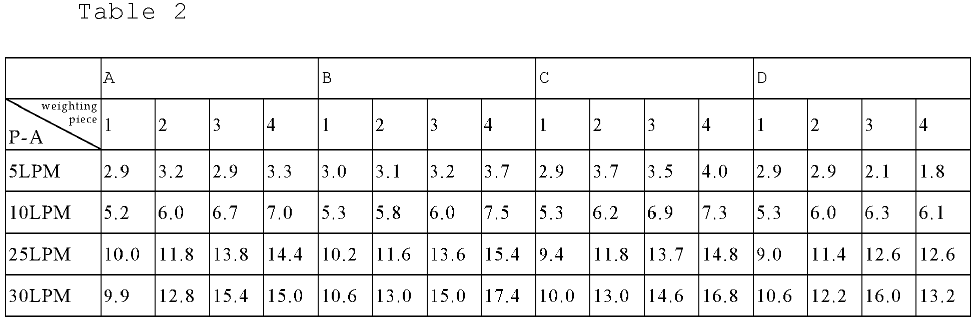

In order to reduce the overall volume of the present oscillating positive expiratory pressure device, the center of gravity of the first weighting piece 32, the rotary pins 312, and the center of gravity of the second weighting piece 33 are configure not to be collinear in the front-rear direction (D1). In addition, to maximize the weight gain efficiency of the first weighting piece 32 and the second weighting piece 33 in balance with the second swing block 314, a ratio between a distance in the front-rear direction (D1) between the center of gravity of the first weighting piece 32 and the axis passing through the rotary pins 312 and a distance in the front-rear direction (D1) between the center of gravity of the second weighting piece 33 and the axis passing the rotary pins 312 is 5:7, which is 10mm:14mm in this embodiment but not limited to this in other embodiments.

The operation of the present oscillating positive expiratory pressure device is as follows: a mouthpiece (not shown) is attached to the input tube 13 of the housing 1 for the user to hold in the mouth. Every time the user exhales, the expiratory airflow passing through the mouthpiece and the input tube 13 will push the second swing block 314 to move upward, making the oscillating unit 3 swing from the closed position where the second swing block 314 blocks the opening 111 to the open position where the second swing block unblocks the opening 111. The curved portion of the inclined enclosing surface 112 is slightly convex curved. When the user's expiratory airflow passes, the Coanda effect will be produced, reducing the air pressure applied against the second swing block 314, thus decreasing the upward acceleration thereof. When the weight of the second swing block 314 and the second weighting piece 33, combined with the Coanda effect of the airflow over the curved portion of the inclined enclosing surface 112, overcomes the expiratory air pressure applied to the second swing block 314, the second swing block 314 descends to block the opening 111 until the user's next expiration. The vibration induced in the expiratory airflow helps loosen the secretions in the trachea of the user, especially in the bronchus, so the user can cough out the secretions. The oscillating swing movement of the oscillating unit 3 between the closed and open positions will continue to vibrate as the user exhales and produce vibrations in the expiratory airflow during operation. It can be appreciated that, since the oscillating frequency of the oscillating unit 3 can be changed by varying the weights of the first weighting piece 32 and the second weighting piece 33, the increase of weight of the first weighting piece 32 and the second weighting piece 33 will reduce the oscillating frequency produced by the oscillating unit 3. In addition, the oscillation frequency of the oscillating unit 3 can also be varied by changing the distance in the front-rear direction (D1) between the center of gravity of the first weighting piece 32 and the axis passing through the rotary pins 312 as well as the distance in the front-rear direction (D1) between the center of gravity of the second weighting piece 33 and the axis passing through the rotary pins 312, depending on the desired oscillating frequency produced by the oscillating unit 3.

It is noted that the first lid 34 and the second lid 35 in the present disclosure are primarily disposed to cover the exposed surface of the first weighting piece 32 and the second weighting piece 33 to prevent them from falling off during operation of the oscillating unit 3, and also provide a flat surface to reduce airflow disturbance and to reduce residual secretions, making it easier to clean. Moreover, in this embodiment, the first weighting piece 32 and the second weighting piece 33 are tightly fitted in the first accommodating groove 313a and the second accommodating groove 314a by the first retaining ribs 313b and the second retaining ribs 314b, respectively. In other embodiments, the amount of first and second retaining ribs 313b and 314b may be reduced. The first and second retaining ribs 313b and 314b may even be omitted entirely in other embodiments if the first accommodating groove 313a and the second accommodating groove 314a are made slightly smaller than the first weighting piece 32 and the second weighting piece 33, such that the first weighting piece 32 and the second weighting piece 33 can tightly fit in the first accommodating groove 313a and the second accommodating groove 314a.

The present oscillating positive expiratory pressure device can also be used in conjunction with a spray device as disclosed in Taiwanese Patent No. I706797. When in use, the input tube 13 of housing 1 can be connected to a mouthpiece (not shown) that communicates with the spray device for the user to hold in the mouth. The present oscillating positive expiratory pressure device is actuated by expiration during the breath cycle. It causes a vibration of the expiratory air pressure, making it easier to cough out secretions in the user's trachea. At the same time, a portion of the user's expiratory airflow is diverted through the mouthpiece to the spray device to temporarily deactivate the spray device. The spray device is activated when the user inhales while suspending the operation of the oscillating positive expiratory pressure device. Therefore, the spray device and the oscillating positive expiratory pressure device operate cyclically when breathing in and out.

In summary, the opening 111 of the present oscillating positive expiratory pressure device is capable of being blocked by the second swing block 314 of the oscillating unit 3 until the user's expiratory airflow lifts the second swing block 314 to unblock the opening 111. When the weight of the second swing block 314 and the second weighting piece 33, combined with the Coanda effect of the airflow over the curved portion of the inclined enclosing surface 112, overcomes the expiratory air pressure applied against the second swing block 314, the second swing block 314 will descend to block the opening 111 again; the vibration of expiratory airflow produced by the oscillating unit 3 can be transmitted to the user's trachea to expand and contract the user's trachea. Through the continuous action of the oscillating unit 3, the secretions adhering to the user's trachea will be loosened such that the user can cough the secretions out.

In the description above, for the purposes of explanation, numerous specific details have been set forth in order to provide a thorough understanding of the embodiment. It will be apparent, however, to one skilled in the art, that one or more other embodiments may be practiced within the scope of the appended claims. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects, and that one or more features or specific details from one embodiment may be practiced together with one or more features or specific details from another embodiment, where appropriate, within the scope of the appended claims.

## Claims

1. An oscillating positive expiratory pressure device including:
a housing (1) including a bottom wall (11) and a surrounding wall (12) that extends upwardly from said bottom wall (11), said bottom wall (11) having an inclined enclosing surface (112) that extends downwardly and that terminates at an opening (111);
a top cover (2) connected to said surrounding wall (12), and cooperating with said bottom wall (11) and said surrounding wall (12) to define an accommodating space (S); and
an oscillating unit (3) swingably connected to said housing (1) and disposed within said accommodating space (S), said oscillating unit (3) including
a swing member (31) that includes
a swing arm (311),
a first swing block (313) connected to one end of said swing arm (311), and
a second swing block (314) connected to another end of said swing arm (311), and adjacent to said opening (111),
a first weighting piece (32) that is carried on said first swing block (313), and
a second weighting piece (33) that is carried on said second swing block (314), said swing arm (311) being swingable to move said second swing block (314) to block and unblock said opening (111);
**characterized in that** *the first swing block(313) is configured and adapted to carry* said first weighting piece (32) of variable weights and the second swing block (314) is configured and adapted to carry said second weighting piece (33) of variable weights to change an oscillating frequency of said oscillating unit (3).

2. The oscillating positive expiratory pressure device as claimed in Claim 1, wherein said second swing block (314) is substantially conical and tapers toward said opening (111).

3. The oscillating positive expiratory pressure device as claimed in Claim 2, wherein:
a cross section of said second swing block (314) along an axis of said second swing block (314) has two opposite sides that form an included angle (α) therebetween ranging between 53 degrees and 73 degrees; and
when said oscillating unit (3) is in the closed position, an included angle (β) defined between each of said opposite sides of the cross section of said second swing block (314) along the axis of said second swing block (314) and said inclined enclosing surface (112) ranges between 10 degrees and 30 degrees.

4. The oscillating positive expiratory pressure device as claimed in Claim 3, wherein said included angle (β) ranges between 16 degrees and 24 degrees.

5. The oscillating positive expiratory pressure device as claimed in any one of Claims 1 to 4, wherein:
said first swing block (313) of said swing arm (311) has a first accommodating groove (313a) accommodating said first weighting piece (32) therein;
said second swing block (314) of said swing arm (311) has a second accommodating groove (314a) accommodating said second weighting piece (33) therein; and
said oscillating unit (3) further includes a first lid (34) closing said first accommodating groove (313a), and a second lid (35) closing said second accommodating groove (314a).

6. The oscillating positive expiratory pressure device as claimed in Claim 5, wherein:
said first swing block (313) further has a plurality of first retaining ribs (313b) disposed in said first accommodating groove (313a) and abutting against said first weighting piece (32); and
said second swing block (314) has a plurality of second retaining ribs (314b) disposed in said second accommodating groove (314a) and abutting against said second weighting piece (33).

7. The oscillating positive expiratory pressure device as claimed in any one of Claims 1 to 6, wherein:
said swing member (31) further includes two rotary pins (312) protruding laterally and respectively from opposite sides of said swing arm (311); and
said bottom wall (11) further has two support brackets (113) holding said two rotary pins (312), respectively.

8. The oscillating positive expiratory pressure device as claimed in Claim 7, wherein:
said swing arm (311) extends along a front-rear direction (D1); and
a ratio between a distance in the front-rear direction (D1) between a center of gravity of said first weighting piece (32) and an axis passing through said rotary pins (312) and a distance in the front-rear direction (D1) between a center of gravity of said second weighting piece (33) and the axis passing through said rotary pins (312) is 5:7.

9. The oscillating positive expiratory pressure device as claimed in any one of Claims 1 to 8, wherein top cover (2) is formed with a plurality of vent holes (21) which occupy less than 50% of the area of said top cover (2).

10. The oscillating positive expiratory pressure device as claimed in any one of Claims 1 to 9, wherein each of said first weighting piece (32) and said second weighting piece (33) has a density greater than 2.

## Revendications

1. Dispositif à pression expiratoire positive oscillante comportant :
un boîtier (1) comportant une paroi inférieure (11) et une paroi périphérique (12) qui s'étend vers le haut à partir de ladite paroi inférieure (11), ladite paroi inférieure (11) ayant une surface d'enceinte inclinée (112) qui s'étend vers le bas et qui se termine au niveau d'une ouverture (111) ;
un couvercle supérieur (2) relié à ladite paroi périphérique (12), et coopérant avec ladite paroi inférieure (11) et ladite paroi périphérique (12) pour définir un espace de réception (S) ; et
une unité d'oscillation (3) reliée de manière pivotante audit boîtier (1) et disposée à l'intérieur dudit espace de réception (S), ladite unité d'oscillation (3) comportant
un élément oscillant (31) qui comporte
un bras oscillant (311),
un premier bloc oscillant (313) relié à une extrémité dudit bras oscillant (311), et
un second bloc oscillant (314) relié à une autre extrémité dudit bras oscillant (311), et adjacent à ladite ouverture (111),
une première pièce de lestage (32) qui est portée sur ledit premier bloc oscillant (313), et
une seconde pièce de lestage (33) qui est portée sur ledit second bloc oscillant (314), ledit bras oscillant (311) pouvant osciller pour déplacer ledit second bloc oscillant (314) pour bloquer et débloquer ladite ouverture (111) ;
**caractérisé en ce que** *le premier bloc oscillant (313) est conçu et adapté pour porter* ladite première pièce de lestage (32) de poids variables et le second bloc oscillant (314) est conçu et adapté pour porter ladite seconde pièce de lestage (33) de poids variables pour changer une fréquence d'oscillation de ladite unité d'oscillation (3).

2. Dispositif à pression expiratoire positive oscillante selon la revendication 1, dans lequel ledit second bloc oscillant (314) est sensiblement conique et s'effile vers ladite ouverture (111).

3. Dispositif à pression expiratoire positive oscillante selon la revendication 2, dans lequel :
une section transversale dudit second bloc oscillant (314) le long d'un axe dudit second bloc oscillant (314) a deux côtés opposés qui forment un angle inclus (α) entre eux compris entre 53 degrés et 73 degrés ; et
lorsque ladite unité d'oscillation (3) est dans la position fermée, un angle inclus (β) défini entre chacun desdits côtés opposés de la section transversale dudit second bloc oscillant (314) le long de l'axe dudit second bloc oscillant (314) et ladite surface d'enceinte inclinée (112) est compris entre 10 degrés et 30 degrés.

4. Dispositif à pression expiratoire positive oscillante selon la revendication 3, dans lequel ledit angle inclus (β) est compris entre 16 degrés et 24 degrés.

5. Dispositif à pression expiratoire positive oscillante selon l'une quelconque des revendications 1 à 4, dans lequel :
ledit premier bloc oscillant (313) dudit bras oscillant (311) a une première rainure de réception (313a) recevant ladite première pièce de lestage (32) en son sein ;
ledit second bloc oscillant (314) dudit bras oscillant (311) a une seconde rainure de réception (314a) recevant ladite seconde pièce de lestage (33) en son sein ; et
ladite unité d'oscillation (3) comporte en outre un premier couvercle (34) fermant ladite première rainure de réception (313a), et un second couvercle (35) fermant ladite seconde rainure de réception (314a).

6. Dispositif à pression expiratoire positive oscillante selon la revendication 5, dans lequel :
ledit premier bloc oscillant (313) présente en outre une pluralité de premières nervures de retenue (313b) disposées dans ladite première rainure de réception (313a) et venant en butée contre ladite première pièce de lestage (32) ; et
ledit second bloc oscillant (314) a une pluralité de secondes nervures de retenue (314b) disposées dans ladite seconde rainure de réception (314a) et venant en butée contre ladite seconde pièce de lestage (33).

7. Dispositif à pression expiratoire positive oscillante selon l'une quelconque des revendications 1 à 6, dans lequel :
ledit élément oscillant (31) comporte en outre deux broches rotatives (312) faisant saillie latéralement et respectivement depuis des côtés opposés dudit bras oscillant (311) ; et
ladite paroi inférieure (11) a en outre deux supports de soutien (113) maintenant lesdites deux broches rotatives (312), respectivement.

8. Dispositif à pression expiratoire positive oscillante selon la revendication 7, dans lequel :
ledit bras oscillant (311) s'étend le long d'une direction avant-arrière (D1) ; et
un rapport entre une distance dans le sens avant-arrière (D1) entre un centre de gravité de ladite première pièce de lestage (32) et un axe passant à travers lesdites broches rotatives (312) et une distance dans le sens avant-arrière (D1) entre un centre de gravité de ladite seconde pièce de lestage (33) et l'axe passant à travers lesdites broches rotatives (312) est de 5:7.

9. Dispositif à pression expiratoire positive oscillante selon l'une quelconque des revendications 1 à 8, dans lequel le couvercle supérieur (2) est formé avec une pluralité de trous d'évent (21) qui occupent moins de 50 % de la surface dudit couvercle supérieur (2).

10. Dispositif à pression expiratoire positive oscillante selon l'une quelconque des revendications 1 à 9, dans lequel chacune de ladite première pièce de lestage (32) et de ladite seconde pièce de lestage (33) a une densité supérieure à 2.

## Patentansprüche

1. Eine oszillierende Vorrichtung für positiven exspiratorischen Druck, einschließlich:
eines Gehäuses (1) einschließlich einer Bodenwand (11) und einer umgebenden Wand (12), die sich von der Bodenwand (11) nach oben erstreckt, wobei die Bodenwand (11) eine geneigte umschließende Oberfläche (112) aufweist, die sich nach unten erstreckt und an einer Öffnung (111) endet;
eine obere Abdeckung (2), die mit der umgebenden Wand (12) verbunden ist und mit der Bodenwand (11) und der umgebenden Wand (12) zusammenwirkt, um einen Aufnahmeraum (S) zu definieren; und
eine oszillierende Einheit (3), die mit dem Gehäuse (1) schwenkbar verbunden ist und innerhalb des Aufnahmeraums (S) angeordnet ist, die oszillierende Einheit (3) einschließlich:
eines Schwenkelements (31), das einschließt:
einen Schwenkarm (311),
einen ersten Schwenkblock (313), der mit einem Ende des Schwenkarms (311) verbunden ist, und
einen zweiten Schwenkblock (314), der mit einem anderen Ende des Schwenkarms (311) verbunden ist und an die Öffnung (111) angrenzt,
eines ersten Beschwerungsstücks (32), das auf dem ersten Schwenkblock (313) getragen wird, und
eines zweiten Beschwerungsstücks (33), das auf dem zweiten Schwenkblock (314) getragen wird, wobei der Schwenkarm (311) schwenkbar ist, um den zweiten Schwenkblock (314) zu bewegen, um die Öffnung (111) zu blockieren und freizugeben;
**dadurch gekennzeichnet, dass** der erste Schwenkblock (313) konfiguriert und angepasst ist, um das erste Beschwerungsstück (32) mit variablen Beschwerungen zu tragen und der zweite Schwenkblock (314) konfiguriert und angepasst ist, um das zweite Beschwerungsstück (33) mit variablen Beschwerungen zu tragen, um eine Oszillationsfrequenz der oszillierenden Einheit (3) zu ändern.

2. Die oszillierende Vorrichtung für positiven exspiratorischen Druck nach Anspruch 1, wobei der zweite Schwenkblock (314) im Wesentlichen konisch ist und sich zu der Öffnung (111) hin verjüngt.

3. Die oszillierende Vorrichtung für positiven exspiratorischen Druck nach Anspruch 2, wobei:
ein Querschnitt des zweiten Schwenkblocks (314) entlang einer Achse des zweiten Schwenkblocks (314) zwei gegenüberliegende Seiten aufweist, die einen eingeschlossenen Winkel (α) dazwischen ausbilden, der im Bereich zwischen 53 Grad und 73 Grad liegt; und
wenn die oszillierende Einheit (3) in der geschlossenen Position ist, ein eingeschlossener Winkel (β), der zwischen jeder der gegenüberliegenden Seiten des Querschnitts des zweiten Schwenkblocks (314) entlang der Achse des zweiten Schwenkblocks (314) und der geneigten umschließenden Oberfläche (112) definiert ist, im Bereich zwischen 10 Grad und 30 Grad liegt.

4. Die oszillierende Vorrichtung für positiven exspiratorischen Druck nach Anspruch 3, wobei der eingeschlossene Winkel (β) im Bereich zwischen 16 Grad und 24 Grad liegt.

5. Die oszillierende Vorrichtung für positiven exspiratorischen Druck nach einem der Ansprüche 1 bis 4, wobei:
der erste Schwenkblock (313) des Schwenkarms (311) eine erste Aufnahmenut (313a) aufweist, die das erste Beschwerungsstück (32) darin aufnimmt;
der zweite Schwenkblock (314) des Schwenkarms (311) eine zweite Aufnahmenut (314a) aufweist, die das zweite Beschwerungsstück (33) darin aufnimmt; und
die oszillierende Einheit (3) ferner einen ersten Deckel (34), der die erste Aufnahmenut (313a) schließt, und einen zweiten Deckel (35) einschließt, der die zweite Aufnahmenut (314a) schließt.

6. Die oszillierende Vorrichtung für positiven exspiratorischen Druck nach Anspruch 5, wobei:
der erste Schwenkblock (313) ferner eine Vielzahl von ersten Halterippen (313b) aufweist, die in der ersten Aufnahmenut (313a) angeordnet sind und an dem ersten Beschwerungsstück (32) anliegen; und
der zweite Schwenkblock (314) eine Vielzahl von zweiten Halterippen (314b) aufweist, die in der zweiten Aufnahmenut (314a) angeordnet sind und an dem zweiten Beschwerungsstück (33) anliegen.

7. Die oszillierende Vorrichtung für positiven exspiratorischen Druck nach einem der Ansprüche 1 bis 6, wobei:
das Schwenkelement (31) ferner zwei Drehstifte (312) einschließt, die seitlich und jeweils von gegenüberliegenden Seiten des Schwenkarms (311) vorstehen; und
die Bodenwand (11) ferner zwei Stützhalterungen (113) aufweist, die jeweils die zwei Drehstifte (312) festhalten.

8. Die oszillierende Vorrichtung für positiven exspiratorischen Druck nach Anspruch 7, wobei:
sich der Schwenkarm (311) entlang einer Vor- und Rückrichtung (D1) erstreckt; und
ein Verhältnis zwischen einem Abstand in der Vor-Rückrichtung (D1) zwischen einem Schwerpunkt des ersten Beschwerungsstücks (32) und einer Achse, die durch die Drehstifte (312) verläuft, und einem Abstand in der Vor-Rückrichtung (D1) zwischen einem Schwerpunkt des zweiten Beschwerungsstücks (33) und der Achse, die durch die Drehstifte (312) verläuft, 5 : 7 beträgt.

9. Die oszillierende Vorrichtung für positiven exspiratorischen Druck nach einem der Ansprüche 1 bis 8, wobei die obere Abdeckung (2) mit einer Vielzahl von Entlüftungslöchern (21) ausgebildet ist, die weniger als 50 % der Fläche der oberen Abdeckung (2) einnehmen.

10. Die oszillierende Vorrichtung für positiven exspiratorischen Druck nach einem der Ansprüche 1 bis 9, wobei jedes des ersten Beschwerungsstücks (32) und des zweiten Beschwerungsstücks (33) eine Dichte von mehr als 2 aufweist.
